# EUROPEAN PATENT SPECIFICATION

(11) **EP 3 125 915 B1**
(45) Date of publication and mention of the grant of the patent: **25.11.2020**
(21) Application number: 14736465.7
(22) Date of filing: 03.04.2014
(51) Int. Cl.: A61K 36/899, A61K 38/16, A61K 31/045

(54) **PROCESS FOR PREPARING A POWDERED MIXTURE OF BORNEOL AND PLANT PROTEINS TO BE USED AS DIETARY OR FOOD SUPPLEMENT, AND RELATED DIETARY OR FOOD SUPPLEMENT**
VERFAHREN ZUR HERSTELLUNG EINER PULVERFÖRMIGEN MISCHUNG AUS BORNEOL UND PFLANZENPROTEINEN ZUR VERWENDUNG ALS DIÄTISCHES ODER NAHRUNGSERGÄNZUNGSMITTEL UND ZUGEHÖRIGES DIÄTISCHES ODER NAHRUNGSERGÄNZUNGSMITTEL
PROCÉDÉ DE PRÉPARATION DE MÉLANGE EN POUDRE DE BORNÉOL ET PROTÉINES VÉGÉTALES À UTILISER COMME COMPLÉMENT DIÉTÉTIQUE OU ALIMENTAIRE, ET COMPLÉMENT DIÉTÉTIQUE OU ALIMENTAIRE ASSOCIÉ

(43) Date of publication of application: 08.02.2017
(73) Proprietor: Biosfered S.r.l., 10148 Torino (IT)
(72) Inventor: MAFFEI, Massimo, I-10148 Torino (IT)
(74) Representative: Garavelli, Paolo
(86) International application number: PCT/IT2014/000097
(87) International publication number: WO 2015/151123

(56) References cited:
- DATABASE WPI Week 201343 Thomson Scientific, London, GB; AN 2013-G29434 XP002732378, & CN 102 886 015 A (UNIV BEIJING CHINESE MEDICINE) 23 January 2013 (2013-01-23)
- DATABASE WPI Week 201310 Thomson Scientific, London, GB; AN 2012-Q00002 XP002732379, & CN 102 630 945 A (TONGLU SHANGPINHUI FOOD CO LTD) 15 August 2012 (2012-08-15)
- DATABASE WPI Week 201036 Thomson Scientific, London, GB; AN 2010-F56463 XP002732380, & CN 101 703 541 A (WANG R) 12 May 2010 (2010-05-12)

## Description

The present invention relates to a process to obtain a borneol solution mixed with plant proteins to be used as a dietary supplement with potential effects aimed to facilitate the opening of the brain-blood barrier to several bioactive molecules.

Disclosed is also a dietary or food supplement produced according to the above process.

Blood-brain barrier (BBB) plays a vital role in controlling the exchange of endogenous and exogenous substances between blood and brain to maintain the homeostasis and normal function of central nervous system (CNS). BBB is mainly formed by brain capillary endothelial cells, void of fenestration, of low pinocytosis and tight junctions, which inhibits transcellular passage of molecules across the barrier and restrict the paracellular diffusion of hydrophilic molecules through endothelial junctions. ATP-binding cassette (ABC) proteins, including P-glycoprotein (P-gp), multidrug resistance-associated proteins (Mrps) and the breast cancer resistance proteins (Bcrp), have been verified to contribute to the function of the BBB in preventing the influx of agent from the blood into the brain and facilitate the efflux of compounds from the brain into the blood. BBB is beneficial for the stabilization of the internal environment of nerve cells, but against the permeation of drugs targeting the brain, thus affecting the therapeutic efficacy on many CNS illness. Generally, BBB does not occur uniformly in all parts of the brain. Some parts around the ventricles are accessible to vital dyes and electron-dense tracers. These areas include the area postrema, median eminence, subcommissural organ, pineal gland, subfornical organ, supraoptic crest and neurohypophysis. Cortex, hippocampus, hypothalamus and striatum are always the brain-targeting areas of CNS disorder. Malignant tumor, epilepsy, dementia, cerebral ischemia and other brain diseases have very high rates of disability and mortality. Currently, many drugs are developed to treat such diseases, but they cannot achieve effectively the purpose to control these diseases because many of these drugs cannot pass through the BBB.

Borneol is a simple bicyclic monoterpene extracted from the resin of the plant *Dryobalanops aromatica* Gaertn.f. (Dipterocarpaceae), commonly known as Borneo Camphor, Camphor Tree, Malay Camphor, or Sumatran Camphor. The plant, according to the theory of traditional Chinese medicine, can direct drugs upward to head targeting the brain. Its main constituent, borneol, has been frequently found in many traditional Chinese compound medicine in clinic for the treatment of CNS illness, such as Alzheimer's disease, stroke cerebral ischemia, cerebritis and cerebral edema.

Recent studies also demonstrated that borneol assisted the permeation of drugs across BBB and enhanced their distribution in the brain tissue. Borneol was found to increase the permeability of geniposide extracted from the medicinal plant *Gynostemma pentaphyllum* in hippocampus and hypothalamus regions, while showed no change in cortex and striatum regions by a microdialysis-UPLC-MS technique. Therefore, borneol has different effects in the four brain regions, and interestingly, borneol, in a low dose range of 0.05-2.0 g/kg, increases the delivery of geniposide in the rat brain, but, in a high dose increasing from 2.0 g/kg to 4.0 g/kg, decreased the brain delivery of geniposide. Borneol also improves the absorption of quercetin in mice brain tissue. The tight junction membrane proteins, claudin-5 and occludin, were studied in rat blood-optic nerve barrier after borneol treatment. It was concluded that claudin-5 and occludin translocate within cells of the rat blood-optic nerve barrier after borneol treatment, and this translocation was reversible. Therefore, claudin-5 may play a potential role in permeability of the blood-optic nerve barrier following borneol treatment. To research the content changes of excitatory neurotransmitter and inhibitory neurotransmitter in corpus striatum of rats, borneol was used in combination with diazepam. The content of both the inhibitory neurotransmitters glycine and gamma-aminobutyric acid (GABA) in corpus striatum of rats with borneol increased significantly, compared with diazepam group. Gastrodin is a phenolic compound from the dry tuber of *Gastrodia elata* Blume, a famous restorative food in some areas of China that exerts immunomodulation and anti-hepatocellular carcinoma activity. Compared with the administration of gastrodin alone, gastrodin co-administrated with borneol was rapidly absorbed from the gastrointestinal tract; the peak time of gastrodin in the plasma became shorter (5-15 vs. 30 min); the bioavailability of gastrodigenin in the brain was increased by 33.6-108.8%; and obvious brain-targeting effect was observed. The enhancing effect was attenuated when the time interval between the administration of borneol and gastrodin was longer than 40 min. The results indicate that borneol can accelerate the absorption of gastrodin in the gastrointestinal tract and promote its distribution to the brain. Nimustine is a nitrosourea agent widely used in central Europe and most Asian regions. Borneol was demonstrated to increase the bioavailability of nimustine in the CSF and the increase of concentration of nimustine in the CSF of rabbits confirmed that borneol can improve the permeability of BBB. After oral administration of 15, 30, 90 mg kg⁻¹ of borneol, oral bioavailability of tetramethylpyrazine phosphate (TMPP) in mice plasma was 1.52, 2.21, 2.95 times increased, respectively, than that without borneol, and 1.12, 1.62, 1.93 times increased, respectively, in brain tissue. Borneol significantly enhances the oral absorption of TMPP and the concentration of TMPP in brain tissue. Borneol was also found to significantly improve the bioavailability of carbamazepine, inhibited the metabolism of carbamazepine, and enhanced the 10,11-epoxide carbamazepine passing through blood brain barrier. Borneol appeared to be a prospecting assisting agent with auxo-action on the penetration of cisplatin across BBB in the therapeutics of brain cancer. Recently, borneol showed tissue specific BBB-opening effect associated with its regulation of the ultrastructure of brain tissues and the expression of transporters of ATP-binding cassette (ABC) family: multidrug resistance 1a (Mdrla), multidrug resistance 1b (Mdrlb), multidrug resistance protein 1 (Mrp1). The latter study indicates that borneol should be used in concert with drugs targeting hippocampus or hypothalamus to exert its synergistic effect to the maximum.

Overall, these results indicate the importance of brain borneol concentration in its BBB-opening effects. Moreover, borneol is a promising promoter for oral brain-targeting drug delivery.

Plant proteins have been recognized to be nutritionally equal, if not superior, to animal proteins, and some plant proteins have the added advantages of being hypoallergenic and healthy for human consumption. For example, rice proteins consist of four traditionally classified fractions based on solubility: albumin (water-soluble), globulin (salt-soluble), glutelin (alkaline-soluble), and prolamin (alcohol-soluble). Globulin (about 12%) and glutelin (about 80%) are the major rice protein components. In general, rice protein, like most cereal proteins, is deficient in the essential amino acid lysine, but has excess of the essential sulfur-containing amino acids, cystine and methionine. Depending on the source of protein and the effect of solvent on the products, various processing designs and methods have been developed through the years and, recently, a patent described the use of plant proteins for the drying of phytoextracts.

The present invention provides for a process of combining borneol with specific plant proteins comprising the steps of: (a) preparation of a solution of borneol; (b) mixing of high purity plant proteins with the borneol solution; (c) drying and milling of the plant protein-borneol solution to a powdered combination of proteins and borneol, the so called PB-154.

Chinese patent applications CN 102630945 A, CN 101703541 A, and CN 102886015 A disclose compositions comprising borneol and plant proteins.

The present disclosure provides for a new and stable source of borneol for the preparation of pills, tablets, capsules, emulsions and suspensions.

Owing to the above described properties of borneol, the compositions obtained by the methods of the present invention may be used to obtain a borneol-plant protein mixture that increases borneol stability and improves the permeability of the BBB. The present compositions obtained by the claimed methods may be used as a potential BBB facilitator for the treatment of CNS illness, such as Alzheimer's disease, stroke cerebral ischemia, cerebritis and cerebral edema, depression and insomnia.

The compositions obtained by the claimed methods of the present invention may be used to potentially facilitate the passage of bioactive compounds through the BBB.

The present invention provides for methods of combining borneol with specific cereal or non-cereal proteins in order to obtain a stable mixture that is eventually dried to obtain a powder, the so called PB-154.

Borneol is weighed and dissolved in a solvent, and said solvent comprising at least one of the substances selected from either acetone or ethyl alcohol. The concentration of borneol may vary according to the final use of PB-154. A typical concentration is in the range between 100 and 150 grams per litre of solvent.

This solution is then mixed with plant proteins which come from the cereal group constituted by rice, wheat, maize, oats, barley, millet, and teff. Alternatively, non-cereal proteins can be used which come from the group constituted by buckwheat, amaranth, hemp, chickpeas, peas, beans, broad beans, soya.

The borneol solution is added to the above mentioned plant proteins in a ratio variable from between 1:1 and 1:2.

The plant protein is added to the borneol solution while mixing and the process continues until the mixture forms a compact paste, which is then placed in stainless steel trays and dried at room temperature in a closed cabinet with forced ventilation.

At the end of the drying process, the dried material is extracted from the trays and crushed with a mill. The degree of crushing can vary according to the final use of the powder. In general, using sieves of 0.5-1 mm. The pulverization time depends on the speed of the crushing mill. Subsequently, the ground product can be collected in containers provided with bags and then stored in a cool, dry place away from light.

The validation of the method follows. Preferably, a sample of the pulverized product is extracted and analyzed by chromatography (liquid or gaseous) in combination with mass spectrometry. The analysis on the total content of plant protein and amino acid composition as well as on borneol is then carried out. The experimental values may thus confirm the perfect mixing of the borneol with the protein matrix and indicate the absence of alterations in the chemical composition.

In particular, the process is economical and requires not particularly costly equipment; it consumes a relatively low amount of energy, does not pollute the environment and uses relatively low temperatures (20 to 25 °C) for the mixing and drying phases.

The powdered preparation, the so called PB-154, can be prepared with different borneol concentrations, ranging from 1% to 90% borneol, preferably from 10% to 40% borneol. PB-154 can then be used to prepare tablets, pills, capsules, emulsions or suspensions to be used in dietary supplements.

The bioactive dose of borneol ranges between 1 mg/Kg⁻¹ and 100 mg/Kg⁻¹, according to scientific literature reports.

PB-154 may be used as a potential BBB facilitator for the treatment of several disorders including CNS illness, such as Alzheimer's disease, stroke cerebral ischemia, cerebritis and cerebral edema, depression and insomnia. Moreover, the ability to open BBB can potentially be exploited is association with chemotherapeutic agents that include, but are not limited to, DNA alkylating agents, topoisomerase inhibitors, endoplasmic reticulum stress inducing agents, platinum compounds, antimetabolites, vincalkaloids, taxanes, epothilones, enzyme inhibitors, receptor antagonists, therapeutic antibodies, tyrosine kinase inhibitors, boron radiosensitizers (i.e. velcade), and chemotherapeutic combination therapies.

The use of PB-154 should be between 10 and 60 min before treatment with other molecules, preferably between 20 and 40 min before treatment.

Owing to the above reported properties of borneol, phytochemicals that scantly cross the BBB or that show low ability to be absorbed can be used in association with PB-154. These include for instance but are not limited to: gipenosides, ginsenosides, scutellarein-7-glucuronide, quercetin, gastrodin, salvanic acid, salvianolic acid, hyperforin, akebia saponin D, puerarin, protocatechuic acid, berberine, and thymoquinone. Other molecules that have been shown to be more permeable in the presence of borneol are: nimustine, tetramethylpyrazine phosphate, carbamazepine, cisplatin, midazolam, muscone, pantoprazole, methotrexate, rifampicin, diazepam, and azidothymidine palmitate liposomes. Furthermore, PB-154 can be used to exploit other borneol effects on intercellular tight junction and pinocytosis vesicles in vitro blood-brain barrier model; cerebral ischemia-reperfusion injury; protection of primary rat hepatocytes against exogenous oxidative DNA damage; inhibition of acetylcholine-mediated effects; agonists effect on TRPV3; protection on cortical neurons against oxygen-glucose deprivation/reperfusion; as an enhancer for targeting aprotinin-conjugated PEG-PLGA nanoparticles to the brain, and general promotion of drugs through BBB.

The process according to the invention will be hereinafter described also by means of examples of embodiment. However, it is understood that the process of the invention is not limited to these examples, but both materials mentioned in the examples and the conditions can be modified while remaining within the invention.

### Example 1

### Mixing of borneol with rice proteins

The first step is the chemical characterization of rice protein through essays on the total protein content (Bradford assay, through the use of the dye Coumassie Blue) and amino acid composition through extraction and characterization by liquid chromatography coupled to mass spectrometry. Then borneol is analyzed by gas-chromatography coupled to mass spectrometry and by gas-chromatography coupled to flame ionization detector by using capillary polar columns (e.g., Carbowax).

Having assessed the purity or reagents, a borneol solution is prepared by dissolving 500 grams of borneol in 4.5 liters of ethyl alcohol (95%). The solutions is obtained by stirring the ethanolic solution with a magnetic stirrer.

4.44 Kg of rice protein are then weighed. By using a kneader equipped with a rotating drum and a spiral dough that rotates in the opposite direction to the rotation of the drum, the borneol solution is poured in the drum and the rice protein is dropped slowly into the alcoholic borneol solution. The rotation speed of the drum is 10 revolutions per minute, while the speed of rotation of the spiral is 90 revolutions per minute. Kneading is performed at room temperature (20-22 °C) up to the achievement of a product homogeneity, the mixing time is 15 minutes.

By considering the borneol and the rice protein weight, a 1:1 ratio between the borneol solution and the rice protein is obtained and a final 10% concentration of borneol is achieved.

The dough is then stretched out on stainless steel trays. The drying process continues in a ventilated cabinet for drying at room temperature. The drying time in the case of ethanol is 5 hours.

The dried material is then removed from the stainless steel trays and crushed with a mill. The degree of crushing can vary according to the final use of the powder. In the example, sieves of 0.5 mm are used. The ground product is collected in containers provided with bags and then stored in a cool, dry place away from light.

The next step is the validation of the method. In the example, a sample of the pulverized product is extracted and analyzed by gas-chromatography coupled to mass spectrometry for the identification and by gas-chromatography coupled to a flame ionization detector for the quantification of borneol. By considering the ratio used, a final 100 mg of borneol per gram of PB-154 is expected. Experimental data indicate that the exhaustive extraction of PB-154 shows the presence of 99.8 ± 0.3 mg borneol per gram of PB-154. A total protein content and amino acid composition analysis is also carried out as described above. The experimental values on total proteins are 98.8% ± 1.1, similar to the original protein before the dough. When a gentle extraction with either hexane, diethyl ether, etanol or acetone is performed, only a fraction of the borneol (about 50%) is released from PB-154. These results indicate that borneol binds tightly to the rice protein and that a drastic extraction or and enzymatic process typical of the digestion is required to deliver the compound. Therefore, the results confirm the perfect mixing of borneol with rice protein, indicate the absence of alterations in the chemical composition of the active principle and the higher bioavailabilty of the compound.

### Example 2

### Mixing of borneol with hemp proteins

As in Example 1, the first step is the chemical characterization of hemp protein and amino acid composition through extraction and characterization by liquid chromatography coupled to mass spectrometry. Then borneol is analyzed by gas-chromatography coupled to mass spectrometry and by gas-chromatography coupled to flame ionization detector as in Example 1.

A borneol solution is prepared by dissolving 600 grams of borneol in 5 liters of acetone. The solutions is obtained by stirring the acetone solution with a magnetic stirrer.

9.4 Kg of hemp protein are then weighed. By using a kneader equipped with a rotating drum and a spiral dough as example 1, the borneol solution is poured in the drum and the hemp protein is dropped slowly into the acetone borneol solution. The kneader is kept under constant removal of air from its surrounding environment by using an airflow cabinet. The kneader conditions are as in example 1.

By considering the borneol and the hemp protein weight, a 1:1.9 ratio between the borneol solution and the hemp protein is obtained and a final 6% concentration of borneol is achieved.

The dough is then stretched out on stainless steel trays. The drying process continues in a ventilated cabinet for drying at room temperature. The drying time in the case of acetone is 3 hours.

The dried material is then removed from the stainless steel trays and crushed with a mill. The degree of crushing can vary according to the final use of the powder. In the example, sieves of 0.5 mm are used. The ground product is collected in containers provided with bags and then stored in a cool, dry place away from light.

The next step is the validation of the method. In the example, a sample of the pulverized product is extracted and analyzed as in example 1. By considering the ratio used, a final 60 mg/g borneol is expected. Experimental data show that the exhaustive extraction of PB-154 shows the presence of 59.1 ± 0.6 mg borneol per gram of PB-154. A total protein content and amino acid composition analysis is also carried out as described in example 1. The experimental values on total proteins are 98.6% ± 1.3, similar to the original protein before the dough. When a gentle extraction with either hexane, diethyl ether, etanol or acetone is performed, only a fraction of the borneol (about 47%) is released from PB-154. These results indicate that borneol binds tightly to the hemp protein and that a drastic extraction or and enzymatic process typical of the digestion is required to deliver borneol from the protein moiety. Therefore, the results confirm the perfect mixing of borneol with hemp protein, indicate the absence of alterations in the chemical composition of the active principle and the higher bioavailabilty of the compound.

## Claims

1. Process for mixing borneol with plant proteins in the presence of solvents to produce dietary or food supplements, wherein the plant protein is a cereal protein which originates from at least one of the substances selected from the group consisting of rice, wheat, corn, oats, barley, millet, teff, the process comprising the steps of:
- providing a borneol solution in either acetone or ethyl alcohol;
- mixing the borneol solution with at least one plant protein, said mixing step being conducted by kneading;
- drying the mixture; and
- milling the mixture in order to obtain a powder;
wherein said at least one plant protein comprises at least one of the substances of the group consisting of cereal proteins, and said solvents comprise at least one of the substances selected from either acetone or ethyl alcohol; and
wherein said borneol is added to the plant protein as an ethanolic or acetone solution in a variable ratio between 1:1 and 1:2 [ethanolic or acetone solution : plant protein], a concentration of borneol in the ethanolic or acetone solution ranging between 100 and 150 grams per litre of solution.

2. Process for mixing borneol with plant proteins in the presence of solvents to produce dietary or food supplements, wherein the plant protein is a non-cereal protein which originates from at least one of the substances selected from the group consisting of buckwheat, amaranth, hemp, chickpeas, peas, beans, broad beans, soybeans, the process comprising the steps of:
- providing a borneol solution in either acetone or ethyl alcohol;
- mixing the borneol solution with at least one plant protein, said mixing step being conducted by kneading;
- drying the mixture; and
- milling the mixture in order to obtain a powder;
wherein said at least one plant protein comprises at least one of the substances of the group of non-cereal proteins, and said solvents comprise at least one of the substances selected from either acetone or ethyl alcohol; and
wherein said borneol is added to the plant protein as an ethanolic or acetone solution in a variable ratio between 1:1 and 1:2 [ethanolic or acetone solution : plant protein], a concentration of borneol in the ethanolic or acetone solution ranging between 100 and 150 grams per litre of solution.

## Patentansprüche

1. Verfahren für die Mischung von Borneol mit pflanzlichen Proteinen bei Vorhandensein von Lösungsmitteln für die Herstellung von Diät- oder Nahrungsergänzungsmitteln, in denen das pflanzliche Protein ein Getreideprotein ist, das aus mindestens einem der Stoffe kommt, die aus der Gruppe bestehend aus Reis, Weizen, Mais, Hafer, Gerste, Hirse, Teff gewählt wurden, das Verfahren enthält folgende Phasen:
- Eine Borneollösung in Aceton oder Ethylalkohol vorbereiten;
- die Borneollösung mit mindestens einem pflanzlichen Protein vermischen, die genannte Mischphase erfolgt durch Kneten;
- die Mischung trocknen; und
- die Mischung mahlen, um ein Pulver zu erhalten;
wobei das genannte mindestens eine pflanzliche Protein mindestens einen der Stoffe enthält, die aus der Gruppe bestehend aus Getreideproteinen gewählt wurden, und die genannten Lösungsmittel enthalten mindestens einen der gewählten Stoffe von Aceton oder Ethylalkohol; und
wobei das genannte Borneol zum pflanzlichen Protein als ethanolische Lösung oder Acetonlösung in einem variablen Verhältnis zwischen 1:1 und 1:2 hinzugefügt wird [ethanolische Lösung oder Acetonlösung : pflanzliches Protein], eine Bomeolkonzentration in der ethanolischen Lösung oder in der Acetonlösung variiert zwischen 100 und 150 Gramm pro Liter der Lösung.

2. Verfahren für die Mischung von Borneol mit pflanzlichen Proteinen bei Vorhandensein von Lösungsmitteln für die Herstellung von Diät- oder Nahrungsergänzungsmitteln, in denen das pflanzliche Protein kein Getreideprotein ist, das aus mindestens einem der Stoffe kommt, die aus der Gruppe bestehend aus Buchweizen, Amarant, Hanf, Kichererbsen, Erbsen, Bohnen, Feldbohnen, Soja gewählt wurden, das Verfahren enthält folgende Phasen:
- Eine Borneollösung in Aceton oder Ethylalkohol vorbereiten;
- die Borneollösung mit mindestens einem pflanzlichen Protein vermischen, die genannte Mischphase erfolgt durch Kneten;
- die Mischung trocknen; und
- die Mischung mahlen, um ein Pulver zu erhalten;
wobei das genannte mindestens eine pflanzliche Protein mindestens einen der Stoffe enthält, die aus der Gruppe von Proteinen, die nicht aus Getreideproteinen bestehen, gewählt wurden, und die genannten Lösungsmittel enthalten mindestens einen der gewählten Stoffe von Aceton oder Ethylalkohol; und
wobei das genannte Borneol zum pflanzlichen Protein als ethanolische Lösung oder Acetonlösung in einem variablen Verhältnis zwischen 1:1 und 1:2 hinzugefügt wird [ethanolische Lösung oder Acetonlösung : pflanzliches Protein], eine Borneolkonzentration in der ethanolischen Lösung oder in der Acetonlösung variiert zwischen 100 und 150 Gramm pro Liter der Lösung.

## Revendications

1. Procédé pour le mélange du bornéol avec des protéines végétales en présence de solvants pour produire des suppléments diététiques ou des compléments alimentaires où la protéine végétale est une protéine de céréales appartenant au moins à l'une des substances choisies dans le groupe composé de riz, blé, maïs, avoine, millet, teff ; le procédé comprend les phases suivantes :
- prévoir une solution de bornéol en acétone ou en alcool éthylique ;
- mélanger la solution de bornéol avec au moins une protéine végétale ; cette phase de mélange est réalisée à travers un pétrissage ;
- sécher le mélange ; et
- broyer le mélange pour obtenir une poudre ;
où au moins une protéine végétale comprend l'une des substances choisies dans le groupe composé de protéines de céréales et les solvants comprennent au moins l'une des substances choisies entre l'acétone ou l'alcool éthylique ; et
où le bornéol est ajouté à la protéine végétale comme solution d'éthanol ou solution d'acétone dans un rapport variable entre 1:1 et 1:2 [solution d'éthanol ou d'acétone : protéine végétale], une concentration de bornéol dans la solution d'éthanol ou d'acétone qui varie entre 100 et 150 grammes par litre de solution.

2. Procédé pour le mélange du bornéol avec des protéines végétales en présence de solvants pour produire des suppléments diététiques ou des compléments alimentaires où la protéine végétale est une protéine ne provenant pas de céréales appartenant au moins à l'une des substances choisies dans le groupe composé de blé noir, amarante, chanvre, pois chiches, petits pois, haricots verts, fèves, soja ; la procédure comprend les phases suivantes :
- prévoir une solution de bornéol en acétone ou en alcool éthylique ;
- mélanger la solution de bornéol avec au moins une protéine végétale ; cette phase de mélange est réalisée à travers un pétrissage ;
- sécher le mélange ; et
- broyer le mélange pour obtenir une poudre ;
où au moins une protéine végétale comprend l'une des substances du groupe de protéines ne provenant pas de céréales et les solvants comprennent au moins l'une des substances choisies entre l'acétone ou l'alcool éthylique ; et
où le bornéol est ajouté à la protéine végétale comme solution d'éthanol ou solution d'acétone dans un rapport variable entre 1:1 et 1:2 [solution d'éthanol ou d'acétone : protéine végétale], une concentration de bornéol dans la solution d'éthanol ou d'acétone qui varie entre 100 et 150 grammes par litre de solution.
